# EUROPÄISCHE PATENTSCHRIFT

(11) **EP 3 421 059 B1**
(45) Veröffentlichungstag und Bekanntmachung des Hinweises auf die Patenterteilung: **08.06.2022**
(21) Anmeldenummer: 18178856.3
(22) Anmeldetag: 20.06.2018
(51) Int. Cl.: A61M 1/14, A61M 1/16

(54) **DIALYSATOR MIT VERBESSERTER ANORDNUNG (DYALISATORHALTER UND ANSCHLÜSSE)**
DIALYSER WITH AN IMPROVED ARRANGEMENT (DIALYSER HOLDER AND CONNECTORS)
DIALYSEUR À AGENCEMENT AMÉLIORÉ (SUPPORT DU DIALYSEUR ET CONNECTEURS)

(30) Priorität: 29.06.2017 DE 102017114528
(43) Veröffentlichungstag der Anmeldung: 02.01.2019
(73) Patentinhaber: B. Braun Avitum AG, 34212 Melsungen (DE)
(72) Erfinder: Kai-Uwe, Ritter, 34212 Melsungen (DE)
(74) Vertreter: Winter, Brandl - Partnerschaft mbB

(56) Entgegenhaltungen:
- EP-A1- 0 747 074
- EP-A1- 1 549 364
- WO-A1-2010/121821
- DE-C1- 19 925 297
- US-A1- 2016 074 565

## Beschreibung

Die Erfindung betrifft eine Vorrichtung zur extrakorporalen Blutbehandlung, insbesondere eine Dialysevorrichtung, mit einer Internfluidik für eine Behandlungsflüssigkeit, welche Internfluidik zumindest zwei Flüssigkeitsanschlüsse zum Anschließen eines im Wesentlichen zylinderförmigen Filterelements an die Internfluidik aufweist, um Behandlungsflüssigkeit durch das Filterelement zu leiten, und mit einer Aufnahme zum austauschbaren Halten des Filterelements derart, dass das Filterelement in bestimmungsgemäßer Weise an die Flüssigkeitsanschlüsse der Internfluidik und die extrakorporale Blutleitung anschließbar ist, wobei die Aufnahme zum Halten des Filterelements derart, dass dessen Zylinderlängsachse im Wesentlichen horizontal ausgerichtet ist, ausgebildet ist und das Filterelement in der Aufnahme um seine Zylinderlängsachse derart drehbar ist, dass zumindest einer seiner Flüssigkeitsanschlüsse nach oben weist, um ein Entlüften des Filterelements beim Befüllen zu unterstützen,und/oder dass zum Entleeren zumindest einer seiner Flüssigkeitsanschlüsse nach unten weist, so dass ein Abfließen von Flüssigkeit aus dem Filterelemente erleichtert wird und/oder Flüssigkeit weitgehend vollständig ablaufen kann bzw. abgesaugt werden kann.

Aus dem Stand der Technik sind Vorrichtungen zur extrakorporalen Blutbehandlung allgemein bekannt. Derartige Vorrichtungen oder Maschinen sind für einen dauerhaften Gebrauch konzipiert und werden üblicherweise mit austauschbaren Blutbehandlungseinheiten, die als Single-Use-Artikel (Einmal-Artikel, Wegwerf-Artikel) ausgebildet und bestimmt sind, bestückt. Beispiele für solche Blutbehandlungseinheiten sind Filterelemente sowie insbesondere Dialysatoren.

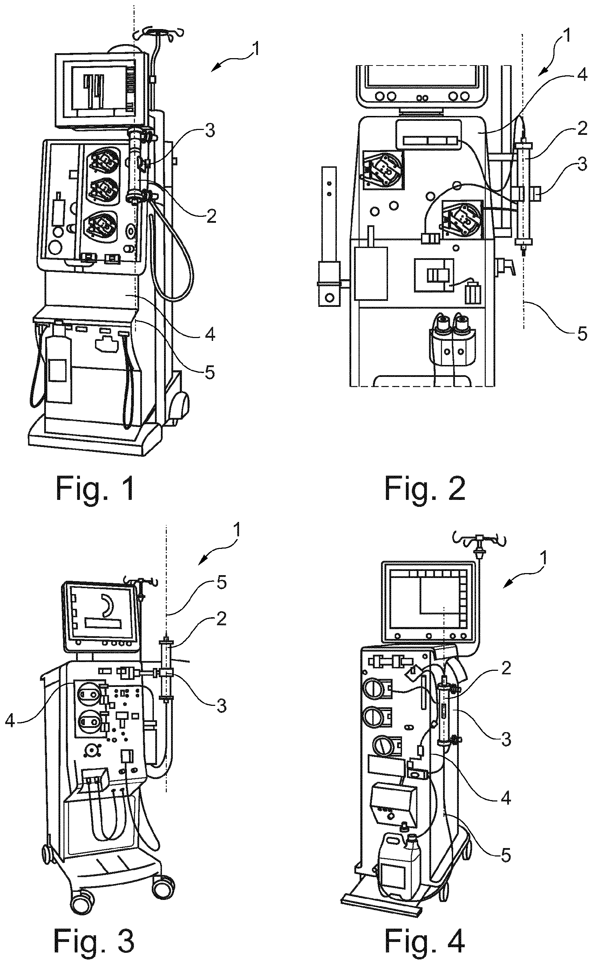

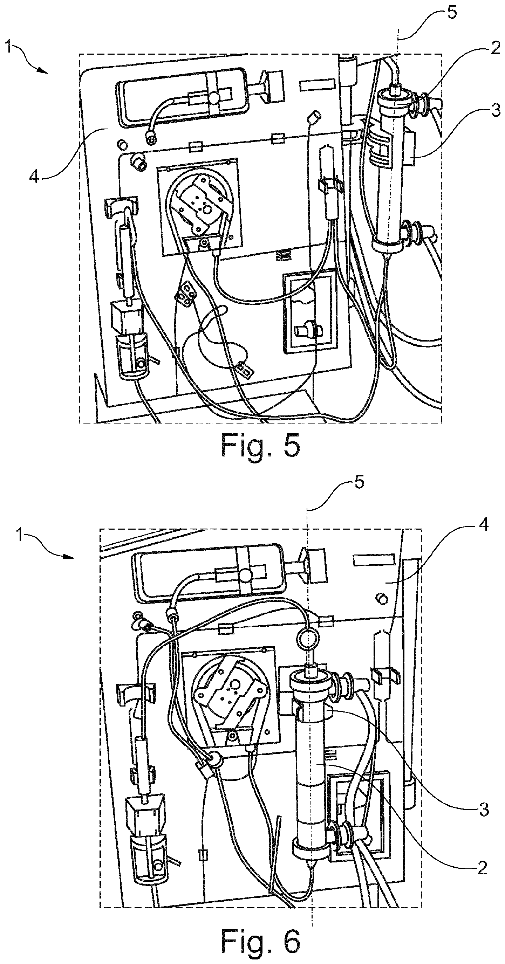

In der Regel besitzen die Blutbehandlungseinheiten eine im Wesentlichen zylinderförmige Gestalt. Bei bekannten Vorrichtungen zur extrakorporalen Blutbehandlung, wie sie in den Figuren 1 bis 6 gezeigt sind, werden oder sind die Blutbehandlungseinheiten für eine Blutbehandlung in einer im Wesentlichen vertikalen Betriebslage an der Vorrichtung angeordnet und mit deren Internfluidik sowie mit der extrakorporalen Blutleitung strömungstechnisch verbunden bzw. verbindbar. Unter einer im Wesentlichen vertikalen Betriebslage ist eine solche Lage oder Position der zylinderförmigen Blutbehandlungseinheit zu verstehen, bei der deren Zylinderlängsachse im Wesentlichen vertikal ausgerichtet ist. Zum Halten der Blutbehandlungseinheit an einer bekannten Vorrichtung weist diese eine Halterung auf, beispielsweise in Form zweier Klemmbacken oder -arme, zwischen die die Blutbehandlungseinheit mit ihrer Zylinderlängsachse in im Wesentlicher vertikaler Position eingebracht und durch Klemmen gehalten werden kann. Die Halterung und die Blutbehandlungseinheit sind derart aufeinander abgestimmt, dass ein Einbringen und ein Entfernen der Blutbehandlungseinheit einfach und unaufwändig sind. Allerdings besteht insbesondere der Nachteil, dass die Blutbehandlungseinheit gegebenenfalls in der Halterung verrutschen kann und eine gewünschte bestimmungsgemäße Position relativ zur Vorrichtung nicht stets sichergestellt ist. Um trotz solcher Positionsvariationen eine sichere und störungsfreie Behandlung gewährleisten zu können, werden die Blutbehandlungseinheiten und die Internfluidik der Vorrichtung mit längeren flexiblen Schlauchsegmenten stömungstechnisch miteinander verbunden. Derart kann zwar ein unbeabsichtigtes Lösen der Schlauchsegmente von der Internfluidik und/oder der Blutbehandlungseinheit vermieden werden, allerdings führen längere Schlauchsegmente zu unerwünschten Temperaturverlusten, sowohl bei in diesen enthaltener Behandlungsflüssigkeit als auch bei in der extrakorporalen Blutleitung enthaltenem Blut. Außerdem führen lange Schlauchsegmente zu einer für eine Bedienperson nur schwer überschaubaren Situation an der Vorrichtung, so dass es zu Fehlanschlüssen sowie zumindest erschwerten Nutzungssituationen kommen kann.

Die US 2016/074565 A1 offenbart eine Vorrichtung zur extrakorporalen Blutbehandlung nach dem Oberbegriff von Anspruch 1.

Ausgehend von dem vorstehend beschriebenen Stand der Technik liegt der vorliegenden Erfindung die Aufgabe zugrunde, die zuvor angeführten Nachteile zu beseitigen, insbesondere eine Vorrichtung zur extrakorporalen Blutbehandlung derart auszubilden, dass ein sicheres und einfaches Austauschen einer Blutbehandlungseinheit gegebenenfalls sogar einhändig möglich ist, die Blutbehandlungseinheit positionssicher an der Vorrichtung gehalten werden kann und Temperaturänderungen und insbesondere Temperaturverluste in an die Blutbehandlungseinheit angeschlossenen Schlauchleitungen möglichst vermieden oder zumindest minimiert werden können.

Nach der Erfindung wird diese Aufgabe gelöst durch eine Vorrichtung zur extrakorporalen Blutbehandlung, insbesondere eine Dialysevorrichtung, mit einer Internfluidik für eine Behandlungsflüssigkeit, insbesondere für eine Dialysierflüssigkeit, welche Internfluidik mit zumindest zwei Flüssigkeitsanschlüssen zum Anschließen eines im Wesentlichen zylinderförmigen Filterelements, insbesondere eines Dialysators, an die Internfluidik aufweist, um Behandlungsflüssigkeit durch das Filterelement zu leiten, und mit einer Aufnahme zum austauschbaren Halten des Filterelements derart, dass das Filterelement in bestimmungsgemäßer Weise an die Flüssigkeitsanschlüsse der Internfluidik und an eine extrakorporale Blutleitung anschließbar ist, wobei die Aufnahme zum Halten des Filterelements derart, dass dessen Zylinderlängsachse im Wesentlichen horizontal ausgerichtet ist, ausgebildet ist und das Filterelement in der Aufnahme um seine Zylinderlängsachse derart drehbar ist, dass zumindest einer seiner Flüssigkeitsanschlüsse nach oben weist, um ein Entlüften des Filterelements beim Befüllen zu unterstützen, und/oder dass zum Entleeren zumindest einer seiner Flüssigkeitsanschlüsse nach unten weist, so dass ein Abfließen von Flüssigkeit aus dem Filterelemente erleichtert wird und/oder Flüssigkeit weitgehend vollständig ablaufen kann bzw. abgesaugt werden kann.

An dieser Stelle sei darauf hingewiesen, dass unter dem Begriff "Internfluidik" jener Teil der Fluid-führenden Leitungsabschnitte zu verstehen ist, der sich stromauf oder stromab zu solchen, vorzugsweise Gehäuse-festen, Anschlüssen anordnet, an die Schlauchleitungen (der Einweg-Bauart) angeschlossen werden, die unmittelbar an den Dialysator gekoppelt sind.

Ein Beispiel für ein an der Vorrichtung anordbares oder angeordnetes Filterelement ist ein üblicher Dialysator, zum Beispiel ein Rohrbündeldialysator. Das Filterelement ist von im Wesentlichen zylinderförmiger Gestalt. Es kann mit auf einen zylinderförmigen Mittenabschnitt beiderseits endseitig angeordneten Abdeckkappen und in beiden axialen Endbereichen des Mittenabschnitts positionierten Anschlüssen für Behandlungsflüssigkeit/Dialysierflüssigkeit und Blut versehen sein. Die Vorrichtung nach der Erfindung kann zur Aufnahme des Filterelements bzw. zur Bestückung damit eingerichtet und geeignet sein. Alternativ kann sie das im Wesentlichen zylinderförmige, austauschbare Filterelement, insbesondere in Form eines Dialysators, umfassen. Das Filterelement kann insbesondere an seinen beiden endseitigen Abschnitten (angrenzend an die Stirnflächen des Zylinders) je einen Blutanschluss und je einen Behandlungsflüssigkeitsanschluss aufweisen, insbesondere einen Blutzulauf, einen Blutablauf, einen Behandlungsflüssigkeitszulauf und einen Behandlungsflüssigkeitsablauf. Der Blutzulauf und der Blutablauf sind an gegenüberliegenden Seiten des Filterelements angeordnet. Auch der Behandlungsflüssigkeitszulauf und der Behandlungsflüssigkeitsablauf sind an gegenüberliegenden Seiten des Filterelements angeordnet. Das Filterelement kann im Gleichstrom und/oder vorzugsweise im Gegenstrom betrieben werden. Ein im Wesentlichen zylinderförmiges Filterelement im Sinne der Erfindung umfasst insbesondere auch ein Filterelement, das eine von der Kreisform abweichende Querschnittsform aufweist, zum Beispiel einen ovalen Querschnitt oder einen vier- oder mehreckigen Querschnitt, auch rechteckigen Querschnitt.

Die Aufnahme kann auch als Halterung bezeichnet werden. Die Vorrichtung kann eine Aufnahme oder mehrere Aufnahmen aufweisen, jeweils für ein Filterelement oder mehrere Filterelemente. Erfindungsgemäß ist die Aufnahme derart ausgebildet, dass ein Filterelement im Wesentlichen horizontal aufgenommen / gehalten / ausgerichtet ist, also quasi auf oder in der Aufnahme ruht und zumindest von unten gestützt ist. Derart kann das Filterelement durch die Schwerkraft unverschiebbar in der Aufnahme und an der Vorrichtung positioniert werden. Im Vergleich zum vorstehend beschriebenen Stand der Technik ermöglicht die Erfindung eine lagebeständigere Anordnung und Halterung des Filterelements an der Vorrichtung. Das Filterelement mit der Internfluidik verbindende Leitungssegmente, wie zum Beispiel Schlauchsegmente, können daher kürzer als beim Stand der Technik, insbesondere möglichst kurz, ausgebildet sein, da keine ungenaue Positionierung oder Lageänderung des Filterelements an der Vorrichtung ausgeglichen werden muss. Infolge der reduzierten Länge der Leitungssegmente können Temperaturschwankungen, insbesondere Abkühlungen, von in diesen strömenden Flüssigkeiten (Dialysierflüssigkeit, Blut) minimiert oder gar vermieden werden. Ein weiterer Vorteil der kurzen Leitungssegmente ist, dass an der Vorrichtung eine bessere Übersichtlichkeit für ein Bedienperson geschaffen werden kann, da zum Beispiel Leitungsschlaufen und -gewirr verhindert werden können.

Eine im Wesentlichen horizontale Lage im Sinne der Erfindung umfasst auch von der genauen horizontalen Lage um einen geringen Winkel abweichende bzw. geneigte Lagen. Ein geringer Winkel ist insbesondere ein solcher Winkel, der ausreichend ist, damit Behandlungsflüssigkeit und/oder Blut im Filterelement, in eine bevorzugte Richtung strömt und besser entfernt werden kann, eine lagestabile Aufnahme des Filterelements jedoch weiterhin sichergestellt ist. Die Neigung des Filterelements ist in jedem Fall geringer als 10°.

Vorzugsweise weist die Internfluidik Flüssigkeitsanschlüsse in Form von Behandlungsflüssigkeitsanschlüssen, insbesondere Dialysierflüssigkeitsanschlüssen, und Spülanschlüssen auf. Insbesondere kann die Internfluidik einen Anschluss zur Zufuhr von Behandlungs-/Dialysierflüssigkeit, einen Anschluss zur Abfuhr von Behandlungs-/Dialysierflüssigkeit sowie zumindest zwei Spülanschlüsse aufweisen. Zumindest einer der oder alle Anschlüsse können in Form von Schnellkupplungen ausgebildet sein, beispielsweise in Form eines Hansen-Anschlusses oder in Form eines Luer-Lock-Anschlusses. Die Anschlüsse können insbesondere fest in ein Gehäuse der Vorrichtung integriert sein, insbesondere in eine Gehäusefront. Nach einer besonders vorteilhaften Ausführungsform weisen die Anschlüsse jeweils eine Kupplung mit automatischem Verschluss auf, so dass die Internfluidik bei nicht verbundenem Filterelement insbesondere leckagefrei abgedichtet und vor Verschmutzung geschützt ist und ein unerwünschtes Austreten von Behandlungsflüssigkeit vermieden werden kann.

Eine Ausführungsform der Erfindung ist dadurch gekennzeichnet, dass die Aufnahme zumindest eine im Wesentlichen horizontal ausgerichtete Kontaktfläche für das Filterelement aufweist. Die Kontaktfläche kann insbesondere als Stützfläche und/oder Klemmfläche ausgebildet sein.

Die Aufnahme kann zumindest eine Klemmvorrichtung zum Halten eines Filterelements umfassen oder in Form einer Klemmvorrichtung ausgebildet sein. Insbesondere kann sie zwei einander gegenüberliegende, vorzugsweise federelastische Klemmarme oder Klemmbacken aufweisen, zwischen denen der Aufnahmeraum für das Filterelement ausgebildet ist. Die Klemmarme bzw. Klemmbacken können zum Beispiel aus Kunststoff oder Edelstahl bestehen. Sie weisen vorzugsweise eine zur Außenkontur des Filterelements passende Innenkontur auf. Außerdem können sie einen Teilumfang des Filterelements umgreifen und derart für eine bestimmungsgemäße und sichere Halterung des Filterelements sorgen.

Alternativ kann die Aufnahme im Wesentlichen gabelförmig ausgebildet sein. Sie kann insbesondere mit zwei nach oben weisenden, einander gegenüberliegenden Gabelarmen versehen sein, zwischen denen der vorzugsweise nach oben offene Aufnahmeraum für das Filterelement ausgebildet ist. Auf eine solche gabelförmige Aufnahme kann das Filterelement besonders einfach aufgelegt werden und befindet sich dennoch stets in bestimmter Lage relativ zur Vorrichtung.

Die Aufnahme ist im Sinne der Erfindung vorzugsweise derart ausgebildet, dass Filterelemente unterschiedlicher Länge und/oder Querschnittsbreite / Durchmesser darin aufgenommen und gehalten werden können. Dies wird nach der Erfindung insbesondere durch Vorsehen einer zu einer Seite hin offenen Aufnahme mit sich in Richtung ihrer Öffnung stetig erweiterten Weite oder durch Vorsehen entsprechend geformter federelastischer Klemmarme oder Klemmbacken erzielt.

Erfindungsgemäß ist die Aufnahme derart ausgebildet, dass das Filterelement darin zwar an definierter Stelle an der Vorrichtung gehalten, jedoch um seine Längsachse drehbar ist. Auf diese Weise kann das Filterelement in unterschiedlichen Drehstellungen in der Aufnahme platziert werden / sein: Zum Beispiel kann es so positioniert sein, dass zumindest einer seiner Flüssigkeitsanschlüsse nach oben weist, so dass ein Entlüften des Filterelements beim Befüllen unterstützt wird. Außerdem kann das Filterelement zum Entleeren derart gedreht werden oder positioniert sein, dass zumindest einer seiner Flüssigkeitsanschlüsse nach unten weist, so dass ein Abfließen von Flüssigkeit aus dem Filterelemente erleichtert wird und/oder Flüssigkeit weitgehend vollständig ablaufen kann bzw. abgesaugt werden kann. Unabhängig von der Lage im Hinblick auf eine Rotation um seine Längsachse ist das Filterelement nach der Erfindung durch die von unten auf dieses wirkende Aufnahme immer lagefixiert relativ zur Vorrichtung daran gehalten.

Nach einer Ausführungsform kann die Aufnahme an der Vorrichtung schwenkbar sein, insbesondere um eine horizontale Achse schwenkbar sein. Diese ist vorzugsweise im Wesentlichen orthogonal zur Längsachse des Filterelements ausgerichtet. Der Winkelbereich, in dem die Aufnahme schwenkbar ist, kann zum Beispiel in einem Bereich von ca. ± 5°, vorzugsweise von ca. ± 2°, bevorzugter von ca. ± 1° und besonders bevorzugt von ca. ± 0,5° liegen. Die Aufnahme kann von der Horizontalen in nur eine oder in beide Richtung schwenkbar sein, wobei die Horizontale als Schwenkwinkel von 0° definiert ist. Durch eine derart schwenkbare Aufnahme kann der Neigungswinkel der im Wesentlichen horizontal angeordneten Längsachse des Filterelements eingestellt und so die Durchströmungscharakteristik des Filterelements variiert werden. Zum Beispiel kann es zum Befüllen und/oder Entleeren geneigt und während einer Behandlung horizontal positioniert werden.

Der Halter kann zusätzlich auch vorsehen, den Dialysator in eine Vertikale Position zu bringen. Dies kann erforderlich sein, falls Dialysatoren zum Einsatz kommen sollen, die ein Befüllen mit Flüssigkeit in horizontaler Lage, oder die Therapie in horizontaler Lage nicht unterstützen würden

Die Aufnahme kann außerdem mit einem Antrieb gekoppelt sein, um diese zum Verschwenken um die Horizontale anzutreiben. Derart kann die Neigungspositionierung des Filterelements automatisiert und maschinengesteuert erfolgen. Außerdem kann die Aufnahme in die oder gegen die Rotationsrichtung um die Horizontale vorgespannt sein. Dies kann insbesondere mittels eines Federelements oder Ähnlichem bewirkt sein. Die Vorspannung kann zum Beispiel derart wirken, dass aus einer geneigten Stellung eine Rückstellung des Filterelements in die Horizontal erfolgt, so dass diese nicht geneigte Position bevorzugt eingenommen wird, sobald das Filterelement nicht mehr in eine geneigte Position gedrängt wird.

Bei einer Ausführungsform der Erfindung kann die Vorrichtung flexible Leitungsabschnitte zum Verbinden der Flüssigkeitsanschlüsse der Internfluidik mit den Blutanschlüssen und den Behandlungsflüssigkeitsanschlüssen des Filterelements umfassen. Die Flüssigkeitsanschlüsse der Internfluidik können mit den flexiblen Leitungsabschnitten verbunden oder verbindbar sein. Solche flexiblen Leitungsabschnitte oder -segmente können insbesondere in Form von an sich auf dem Gebiet der extrakorporalen Blutbehandlung bekannten Schlauchleitungen ausgebildet sein.

Es ist von besonderem Vorteil, wenn die Flüssigkeitsanschlüsse der Internfluidik derart relativ zum Filterelement positioniert sind, und die Länge der mit dem Filterelement zu verbindenden flexiblen Leitungsabschnitte jeweils derart beschaffen ist, dass ein bestimmungsgemäßer Anschluss des Filterelements sichergestellt ist, ein nicht bestimmungsgemäßer Anschluss des Filterelements infolge einer unzureichenden Länge der flexiblen Leitungsabschnitte unmöglich ist. Alternativ oder zusätzlich ist es von besonderem Vorteil, wenn die Flüssigkeitsanschlüsse der Internfluidik derart relativ zum Filterelement positioniert sind, und die Länge der mit dem Filterelement zu verbindenden flexiblen Leitungsabschnitte jeweils derart beschaffen ist, dass eine bestimmungsgemäße Verbindung der Flüssigkeitsanschlüsse miteinander bei einem Spülen der Internfluidik sichergestellt ist, jedoch ein nicht bestimmungsgemäßes Verbinden der Flüssigkeitsanschlüsse miteinander beim Spülen der Internfluidik infolge einer unzureichenden Länge der flexiblen Leitungsabschnitte unmöglich ist. Dies erhöht in vorteilhafter Weise die Bedienungssicherheit der Vorrichtung und vermeidet mögliche Behandlungsfehler in Folge eines fehlerhaften Anschlusses des Filterelements an die Vorrichtung. Zum Beispiel sind die Behandlungsflüssigkeitsanschlüsse der Internfluidik vorzugsweise in einer Gehäusefront der Vorrichtung angeordnet. Außerdem können Spülanschlüsse in einer Gehäusefront der Vorrichtung angeordnet sein. Vorzugsweise sind die Behandlungsflüssigkeitsanschlüsse der Internfluidik in der Nähe der entsprechenden Anschlüsse des Filterelements platziert. Die Spülanschlüsse sind vorzugsweise in der Nähe der Behandlungsflüssigkeitsanschlüsse der Internfluidik angeordnet und relativ weit voneinander beabstandet. Es ist besonders vorteilhaft, wenn der Abstand zwischen den Spülanschlüssen und dem bei einem Spülvorgang damit jeweils fluidisch zu verbindenden Behandlungsflüssigkeitsanschluss kleiner ist als der Abstand zwischen zwei Spülanschlüssen. Vorzugsweise sind die Spülanschlüsse so weit voneinander entfernt, dass ein Verstecken, also ein fehlerhaftes Verbinden von Spülanschluss und Behandlungsflüssigkeitsanschluss, nicht möglich ist. Dafür ist außerdem wesentlich, dass die Länge von Leitungssegmenten zur strömungstechnischen Verbindung der Internfluidik mit dem Filterelement sowie der Spülanschlüsse der Internfluidik mit den Behandlungsflüssigkeitsanschlüssen der Internfluidik derart bemessen ist, dass keine falsche Verbindung erstellt werden kann, also dass die Leitungssegmente ausreichend kurz sind. Beispielsweise können die Leitungssegmente eine beliebige Länge zwischen etwa 10 cm und etwa 30 cm aufweisen.

Zusammenfassend kann man sagen, dass durch die Erfindung insbesondere die folgenden Vorteile und Verbesserungen erzielt werden können:
- Deutlich verbessertes Handling, der Dialysator wird an der Front ein geklickt und rutscht nicht mehr nach unten
- Einhändiges Aufrüsten des Dialysators möglich
- Sehr kurze Blutleitungen, dadurch verringertes Blutvolumen im Schlauch
- Verbesserte Biokompatibilität
- geringere Kosten des Blutschlauchs
- sehr einfache und kostengünstige Konstruktion des Dialysatorhalters möglich
- Geringer Temperatur-/Energieverlust an den DF-Leitungen
- verbesserte Temperaturgenauigkeit am Dialysator
- Verkürzte Desinfektionszeit
- Weniger Raumaufheizung
- Energieeinsparung

Die Erfindung sowie Beispiele zum Stand der Technik werden in den angehängten Figuren gezeigt.
Fig. 1 eine Ansicht auf eine bekannte Vorrichtung zur extrakorporalen Blutbehandlung,
Fig. 2 eine Ansicht auf eine weitere bekannte Vorrichtung zur extrakorporalen Blutbehandlung,
Fig. 3 eine Ansicht auf eine weitere bekannte Vorrichtung zur extrakorporalen Blutbehandlung,
Fig. 4 eine Ansicht auf eine weitere bekannte Vorrichtung zur extrakorporalen Blutbehandlung,
Fig. 5 eine Ansicht auf eine weitere bekannte Vorrichtung zur extrakorporalen Blutbehandlung,
Fig. 6 eine Ansicht auf eine weitere bekannte Vorrichtung zur extrakorporalen Blutbehandlung,
Fig. 7 eine schematisch Aufsicht auf eine Gehäusefront einer Vorrichtung nach der Erfindung,
Fig. 8 eine schematische Teilansicht einer erfindungsgemäßen Vorrichtung mit aufgenommenem Filterelement während einer Therapie bzw. eines Primings,
Fig. 9 eine schematische Teilansicht einer erfindungsgemäßen Vorrichtung mit aufgenommenem Filterelement während einer Entleerung,
Fig. 10 eine schematische Teilansicht einer erfindungsgemäßen Vorrichtung während einer Desinfektion,
Fig. 11 eine schematische Teilansicht einer erfindungsgemäßen Vorrichtung mit aufgenommenem Filterelement mit einer ersten Anordnung von Flüssigkeitsanschlüssen an der Vorrichtung im Rahmen der Erfindung,
Fig. 12 eine schematische Teilansicht einer erfindungsgemäßen Vorrichtung mit aufgenommenem Filterelement mit einer zweiten Anordnung von Flüssigkeitsanschlüssen an der Vorrichtung im Rahmen der Erfindung,
und
Fig. 13 eine schematische einer erfindungsgemäßen Vorrichtung mit aufgenommenem Filterelement mit einer dritten Anordnung von Flüssigkeitsanschlüssen an der Vorrichtung im Rahmen der Erfindung.

Die Figuren 1 bis 6 zeigen jeweils eine Dialysemaschine 1 nach dem Stand der Technik als Beispiel für eine Vorrichtung 1 zur extrakorporalen Blutbehandlung mit einem austauschbar daran angeordneten und mit einer in den Figuren nicht gezeigten Internfluidik strömungstechnisch verbundenen Filterelement 2 in Form eines Dialysators 2. Bei allen gezeigten bekannten Vorrichtungen 1 ist der Dialysator 2 jeweils im Wesentlichen vertikal mittels einer Aufnahme 3 an einem Gehäuse 4 der Vorrichtung 1 angeordnet, derart, dass seine in die Figuren eingezeichnete Längsachse 5 im Wesentlichen vertikal ausgerichtet ist.

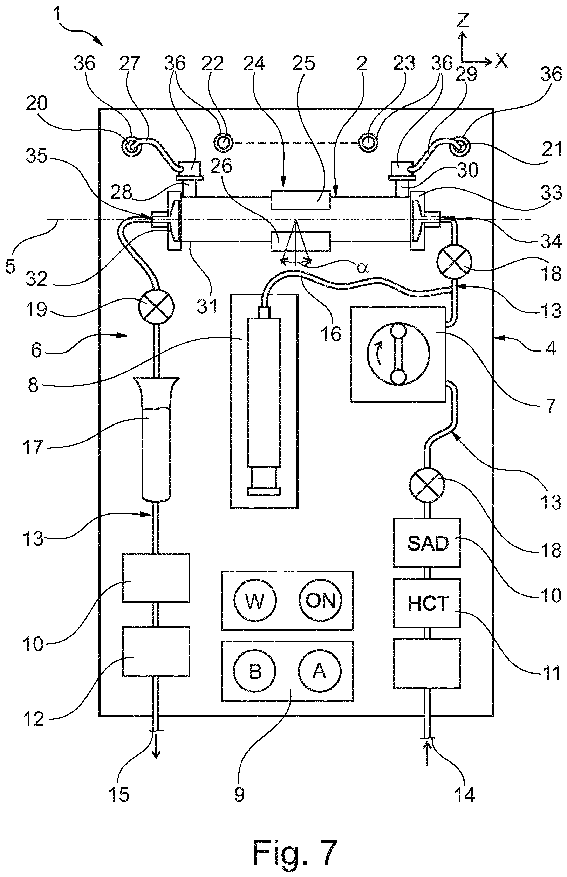

Fig. 7 zeigt in einer schematischen Aufsicht eine Anordnung des Filterelements / Dialysators 2 an einer Front 6 eines Gehäuses 4 einer Vorrichtung 1 nach der Erfindung. In der Figur sind die Horizontale X und die Vertikale Z zur besseren Orientierung eingezeichnet. Die Gehäusefront 6 bildet eine Art Bedienfläche oder Bedienraum aus, quasi als Schnittstelle zwischen einem Nutzer / Bediener und der Maschine. Außer dem Dialysator 2 sind an der Front dem Nutzer zugänglich eine Blutpumpe 7, eine Heparinpumpe 8, ein Konzentratköcher 9, ein oder zwei Luftsensoren 10, ein Hematokrit-Sensor 11 sowie eine Schlauchabsperrklemme venös (SAKV), sowie eventuell eine Schlauchabsperrklemme arteriell (SAKA)12 angeordnet. Außerdem sind in Figur 7 dargestellt eine extrakorporale Blutleitung 13 mit einem arteriellen Anschluss 14 und einem venösen Anschluss 15. Wie in Figur 7 angedeutet ist, ist die extrakorporale Blutleitung 13 in die Blutpumpe 7, hier in Form einer Peristaltikpumpe 7, eingelegt, so dass die Blutpumpe 7 Blut vom arteriellen Anschluss 14 zum venösen Anschluss 15 fördert. Die Heparinpumpe 8 ist strömungstechnisch über eine Heparinleitung 16 mit der extrakorporalen Blutleitung 13 verbunden. Außerdem sind ein Luftblasenfänger 17, zwei arterielle Drucksensoren 18 und ein venöser Drucksensor 19 in die Blutleitung 13 integriert.

Die in Figur 7 nicht gezeigte Internfluidik der Vorrichtung 1 ist hinter der Gehäusefront 6 angeordnet und verborgen. In die Gehäusefront 6 integriert oder eingebaut sind ein Behandlungsflüssigkeitsanschluss 20 zur Zufuhr von Behandlungs-/Dialysierflüssigkeit, ein Behandlungsflüssigkeitsanschluss 21 zur Abfuhr von Behandlungs-/Dialysierflüssigkeit sowie zwei Spülanschlüsse 22, 23. In deren Nähe ist des Weiteren eine Aufnahme 24 für das Filterelement 2 platziert. Diese ist als Klemmeinrichtung ausgebildet und umfasst einen ersten Klemmarm 25 und einen zweiten Klemmarm 26, die zwischen sich einen Aufnahmeraum für das Filterelement 2 ausbilden. Die Klemmarme 25, 26 sind jeweils aus Kunststoff und weisen klauenartige Endabschnitte mit zum Filterelement 2 zugewandten Halte-, Kontakt- oder Stützflächen 38 und in einer Richtung quer zur Längsachse 5 des Filterelements 2 federelastische Eigenschaften auf. Die Kontaktfläche 38 ist im Wesentlichen parallel zur Zylinderlängsachse 5 ausgerichtet. Das Filterelement 2 kann so in an sich bekannter Weise unter elastischer Deformation der Klemmarme 25, 26 in den Aufnahmeraum zwischen diesen eingebracht werden und wird durch deren elastische Rückdeformation in den klauenartigen Endabschnitten gehalten.

Figur 7 zeigt deutlich, dass die Längsachse 5 des in der Aufnahme 24 angeordneten und gehaltenen Filterelements 2 im Wesentlichen horizontal ausgerichtet ist. Infolge der Halterung in den klauenartigen Endabschnitten der Klemmarme 25, 26 auf oder an deren Stützflächen 38 ist es dabei um seine Zylinderlängsachse 5 drehbar. Außerdem ist die Aufnahme 24 um einen Neigungswinkelbereich α um eine Schwenkachse drehbar, die orthogonal zur Längsachse 5 und in Figur 7 orthogonal zur Zeichnungsebene ausgerichtet ist. Der Neigungswinkel α liegt in einem Winkelbereich von ca. ± 5° um die Horizontale, vorzugsweise von ca. ± 2° um die Horizontale, bevorzugter von ca. ± 1° um die Horizontale, besonders bevorzugt von ca. ± 0,5° um die Horizontale, wobei bei einem Neigungswinkel von 0° die Längsachse 5 genau in der Horizontalen ausgerichtet ist.

Das Filterelement 2 weist eine im Wesentlichen zylinderförmige Gestalt auf und umfasst einen zylinderförmigen Mittelabschnitt 31, an dem beiderseits endseitig Endkappen 32, 33 angeordnet sind, die einen Blutzugang 34 und einen Blutabgang 35 ausbilden. Im linken axialen Endabschnitt des Mittelabschnitts 31 ist ein Dialysateingang 28 und im rechten axialen Endabschnitt des Mittelabschnitts 31 ein Dialysatausgang 30 ausgebildet.

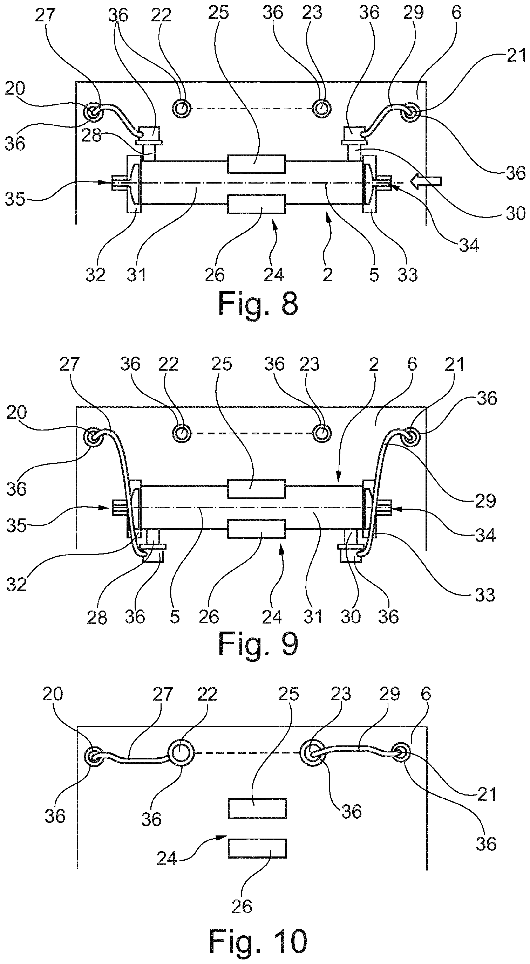

Die Beschreibung der Figuren 8 bis 13 erfolgt beispielhaft anhand einer Dialysevorrichtung 1 mit einem Dialysator 2 als Filterelement und Dialysat als Behandlungsflüssigkeit.

Figur 8 zeigt eine schematische Darstellung eines erfindungsgemäß aufgenommenen Filterelements während einer Therapie bzw. eines Primings. Der Dialysatanschluss 20 zur Zufuhr von Dialysierflüssigkeit, der Dialysatanschluss 21 zur Abfuhr von Dialysierflüssigkeit sowie die beiden Spülanschlüsse 22, 23 sind jeweils in der Nähe und oberhalb des Filterelements 2 angeordnet. Die beiden Spülanschlüsse 22, 23 sind - wie in der Figur angedeutet - intern strömungstechnisch mittels einer Leitung 37, die Bestandteil der Internfluidik ist, miteinander verbunden. Der Dialysatanschluss 20 zur Zufuhr von Dialysierflüssigkeit ist mittels eines ersten Leitungssegments / Anschlussschlauchs / Schlauchsegments 27 strömungstechnisch mit dem Dialysateingang 28 des Filterelements 2 verbunden. Der Dialysatanschluss 21 zur Abfuhr von Dialysierflüssigkeit ist mittels eines zweiten Leitungssegments / Anschlussschlauchs / Schlauchsegments 29 strömungstechnisch mit dem Dialysatausgang 30 des Filterelements 2 verbunden. Die Schlauchsegmente 27, 29 sind mittels Schnellkupplungen 36 auf die jeweiligen Flüssigkeitsanschlüsse 20, 21 aufgesteckt. Ihre Länge und die Anordnung des Dialysatanschlusses 20 zur Zufuhr von Dialysierflüssigkeit, des Dialysatanschlusses 21 zur Abfuhr von Dialysierflüssigkeit sowie der beiden Spülanschlüsse 22, 23 ist dabei derart, dass das Schlauchsegment 27 nur den Dialysatanschluss 20 mit dem Spülanschluss 22 verbinden kann, nicht jedoch den Dialysatanschluss 20 mit dem Spülanschluss 23. In ähnlicher Weise kann das Schlauchsegment 29 nur den Dialysatanschluss 21 mit dem Spülanschluss 23 verbinden, nicht jedoch den Dialysatanschluss 21 mit dem Spülanschluss 22. Das Filterelement 2 wird in Figur 8 von links nach rechts von Dialysat und von rechts nach links von Blut durchströmt, also im Gegenstrom betrieben.

Fig. 9 zeigt eine schematische Darstellung während einer Entleerung des Filterelements. Das Filterelement 2 ist in der mit Bezug auf Figur 8 beschriebenen Weise strömungstechnisch mit der Internfluidik der Vorrichtung verbunden. Es ist allerdings gegenüber der Darstellung der Figur 8 um etwa 180° um seine Längsachse 5 gedreht, so dass der Dialysateingang 28 und der Dialysatausgang 30 nach unten weisen und ein Ausströmen von Dialysat aus dem Filterelement 2 und damit dessen Entleeren erleichtert wird.

Fig. 10 zeigt eine schematische Darstellung einer erfindungsgemäßen Vorrichtung während einer Desinfektion der Internfluidik. Das Filterelement 2 ist von der Internfluidik entkoppelt und aus der Aufnahme 24 entfernt. Das Schlauchsegment 27 ist auf den Spülanschluss 22 aufgesteckt, so dass dieser mit dem Dialysatanschluss 20 verbunden ist. Das Schlauchsegment 29 ist auf den Spülanschluss 23 aufgesteckt, so dass dieser mit dem Dialysatanschluss 21 verbunden ist.

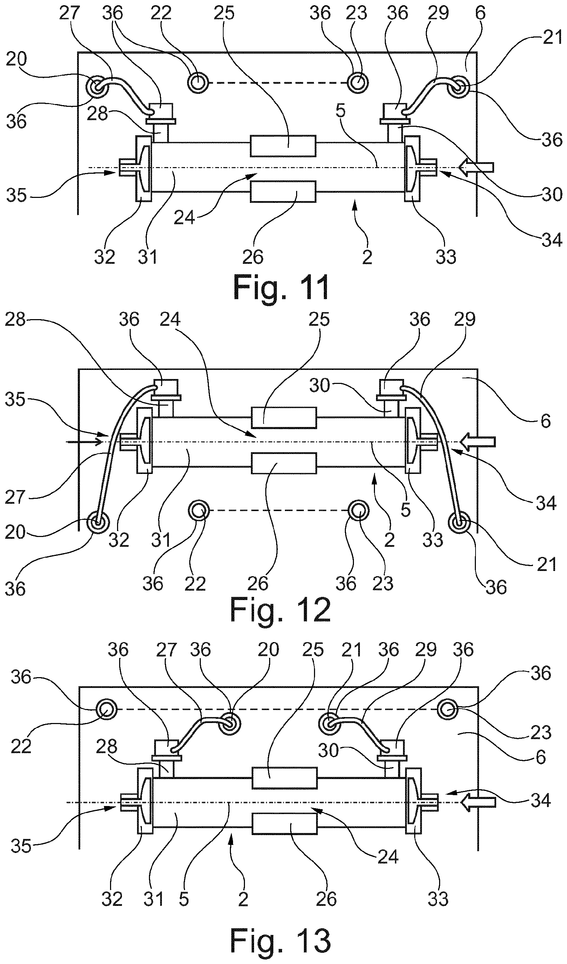

Die Figuren 11, 12 und 13 zeigen jeweils in einer schematischen Darstellung die Anordnung von Flüssigkeitsanschlüssen an der Vorrichtung relativ zum Filterelement 2. Bei der Ausführungsform der Figur 11 sind sowohl die Dialysatanschlüsse 20 und 21 wie auch die Spülanschlüsse 22, 23 oberhalb der Aufnahme 24 angeordnet, siehe auch in der Beschreibung der Figur 8. Eine alternative Anordnung zeigt Figur 12, bei der sowohl die Dialysatanschlüsse 20 und 21 als auch die Spülanschlüsse 22, 23 unterhalb der Aufnahme 24 angeordnet sind. Bei beiden Varianten sind die Spülanschlüsse 22, 23 zwischen den Dialysatanschlüssen 20 und 21 platziert. Figur 13 zeigt eine weitere Variante, bei der sowohl die Dialysatanschlüsse 20 und 21 als auch die Spülanschlüsse 22, 23 oberhalb der Aufnahme 24 angeordnet sind, allerdings abweichend von der Ausführungsform der Figur 11 die Spülanschlüsse 22, 23 außen und die Dialysatanschlüsse 20 und 21 innen zwischen den Spülanschlüssen 22, 23 angeordnet sind. Allen Ausführungsformen ist gemein, dass ein falsches Verbinden der Schlauchsegmente 27, 29, wie vorstehend bereits mit Bezug auf Figur 8 beschrieben wurde, nicht möglich ist.

### Bezugszeichen

- 1: Vorrichtung zur extrakorporalen Blutbehandlung, Dialysemaschine
- 2: Filterelement, Dialysator
- 3: Aufnahme, Halterung
- 4: Gehäuse
- 5: Längsachse
- 6: Gehäusefront, Front
- 7: Blutpumpe
- 8: Heparinpumpe
- 9: Konzentratköcher
- 10: Luftsensor
- 11: Hematokritsensor
- 12: Schlauchabsperrklemme venös
- 12a: Schlauchabsperrklemme arteriell (muss noch benummert werden)
- 13: extrakorporale Blutleitung
- 14: arterieller Anschluss
- 15: venöser Anschluss
- 16: Heparinleitung
- 17: Luftblasenfänger
- 18: arterieller Drucksensor
- 19: venöser Drucksensor
- 20: Behandlungsflüssigkeitsanschluss Zufuhr
- 21: Behandlungsflüssigkeitsanschluss Abfuhr
- 22: Spülanschluss
- 23: Spülanschluss
- 24: Aufnahme
- 25: erster Klemmarm
- 26: zweiter Klemmarm
- 27: Leitungssegment, Schlauchsegment, Anschlussschlauch
- 28: Dialysateingang
- 29: Leitungssegment, Schlauchsegment, Anschlussschlauch
- 30: Dialysatausgang
- 31: Mittelabschnitt
- 32: Endkappe
- 33: Endkappe
- 34: Blutzugang
- 35: Blutabgang
- 36: Schnellkupplung
- 37: Verbindungsleitung
- 38: Kontaktfläche, Stützfläche, Haltefläche
- α: Neigungswinkel der Aufnahme

## Patentansprüche

1. Vorrichtung (1) zur extrakorporalen Blutbehandlung, insbesondere Dialysevorrichtung (1),
mit einer Internfluidik für eine Behandlungsflüssigkeit, insbesondere für eine Dialysierflüssigkeit,
welche Internfluidik zumindest zwei Flüssigkeitsanschlüsse (20, 21, 22, 23) zum Anschließen eines im Wesentlichen zylinderförmigen Filterelements (2), insbesondere eines Dialysators (2), an die Internfluidik aufweist, um Behandlungsflüssigkeit durch das Filterelement (2) zu leiten, und
mit einer Aufnahme (24) zum austauschbaren Halten des Filterelements (2) derart, dass das Filterelement (2) in bestimmungsgemäßer Weise an die Flüssigkeitsanschlüsse (20, 21, 22, 23) der Internfluidik und an eine extrakorporale Blutleitung (13) anschließbar ist,
wobei die Aufnahme (24) zum Halten des Filterelements (2) derart, dass dessen Zylinderlängsachse (5) im Wesentlichen horizontal ausgerichtet ist, ausgebildet ist und das Filterelement (2) in der Aufnahme (24) um seine Zylinderlängsachse (5) derart drehbar ist, dass zumindest einer seiner Flüssigkeitsanschlüsse nach oben weist, um ein Entlüften des Filterelements beim Befüllen zu unterstützen, und/oder dass zum Entleeren zumindest einer seiner Flüssigkeitsanschlüsse nach unten weist, so dass ein Abfließen von Flüssigkeit aus dem Filterelemente erleichtert wird und/oder Flüssigkeit weitgehend vollständig ablaufen kann bzw. abgesaugt werden kann.

2. Vorrichtung (1) nach Anspruch 1, **dadurch gekennzeichnet, dass** die Aufnahme (24) zumindest eine im Wesentlichen horizontal ausgerichtete Kontaktfläche (38) für das Filterelement (2) aufweist.

3. Vorrichtung (1) nach einem der vorstehenden Ansprüche, **dadurch gekennzeichnet, dass** die Aufnahme (24) an der Vorrichtung (1) um eine horizontale Achse (5) schwenkbar ist, insbesondere in einem Winkelbereich α von ca. ± 5° um die Horizontale, vorzugsweise von ca. ± 2° um die Horizontale, bevorzugter von ca. ± 1° um die Horizontale, besonders bevorzugt von ca. ± 0,5° um die Horizontale.

4. Vorrichtung (1) nach Anspruch 3, **dadurch gekennzeichnet, dass** die Aufnahme (24) mit einem Antrieb gekoppelt ist, zum angetriebenen Verschwenken im Winkelbereich α um die Horizontale.

5. Vorrichtung (1) nach Anspruch 3 oder 4, **dadurch gekennzeichnet, dass** die Aufnahme (24) in die oder gegen die Schwenkrichtung vorgespannt ist, insbesondere mittels eines Federelements.

6. Vorrichtung (1) nach einem der vorstehenden Ansprüche, **dadurch gekennzeichnet, dass** die Aufnahme (24) zumindest eine Klemmvorrichtung (25, 26) zum Halten eines Filterelements (2) umfasst.

7. Vorrichtung (1) nach einem der vorstehenden Ansprüche, des Weiteren umfassend das im Wesentlichen zylinderförmige, austauschbare Filterelement (2), insbesondere einen Dialysator (2).

8. Vorrichtung (1) nach Anspruch 7, **dadurch gekennzeichnet, dass** das Filterelement (2) seinen an beiden endseitigen Abschnitten je einen Blutanschluss (34, 35) und je einen Behandlungsflüssigkeitsanschluss (28, 30) aufweist.

9. Vorrichtung (1) nach einem der vorstehenden Ansprüche, des Weiteren umfassend flexible Leitungsabschnitte (27, 29) zum Verbinden der Flüssigkeitsanschlüsse (20, 21, 22, 23) der Internfluidik mit Blutanschlüssen (34, 35) und Behandlungsflüssigkeitsanschlüssen (28, 30) des Filterelements (2).

10. Vorrichtung (1) nach Anspruch 9, **dadurch gekennzeichnet, dass** die Flüssigkeitsanschlüsse (20, 21, 22, 23) der Internfluidik mit den flexiblen Leitungsabschnitten (27, 29) verbunden oder verbindbar sind und die Flüssigkeitsanschlüsse (20, 21, 22, 23) der Internfluidik derart relativ zum Filterelement (2) positioniert sind, und die Länge der mit dem Filterelement (2) zu verbindenden flexiblen Leitungsabschnitte (27, 29) jeweils derart beschaffen ist, dass ein bestimmungsgemäßer Anschluss des Filterelements (2) an die Internfluidik und/oder eine bestimmungsgemäße Verbindung der Flüssigkeitsanschlüsse (20, 21, 22, 23) miteinander bei einem Spülen der Internfluidik sichergestellt ist, jedoch ein nicht bestimmungsgemäßer Anschluss des Filterelements (2) an die Internfluidik und/oder ein nicht bestimmungsgemäßes Verbinden der Flüssigkeitsanschlüsse (20, 21, 22, 23) miteinander beim Spülen der Internfluidik infolge einer unzureichenden Länge der flexiblen Leitungsabschnitte (27, 29) unmöglich ist.

## Claims

1. A device (1) for extracorporeal blood treatment, in particular a dialysis device (1),
comprising an internal fluidic system for a treatment liquid, in particular for a dialysis liquid,
said internal fluidic system having at least two liquid connectors (20, 21, 22, 23) for connecting a substantially cylindrical filter element (2), in particular a dialyzer (2), to the internal fluidic system for passing treatment liquid through the filter element (2), and
comprising a mounting (24) for exchangeably holding the filter element (2) in such a way that the filter element (2) can be connected to the liquid connectors (20, 21, 22, 23) of the internal fluidic system and to an extracorporeal blood line (13) in the intended manner,
wherein the mounting (24) for holding the filter element (2) is designed in such a way that its longitudinal cylinder axis (5) is substantially horizontally aligned and the filter element (2) is rotatable in the mounting (24) about its longitudinal cylinder axis (5) in such a way that at least one of its liquid connectors points upwards to assist venting of the filter element during filling, and/or that at least one of its liquid connectors points downwards for draining so that draining of liquid from the filter element is facilitated and/or liquid can run off or be sucked off largely completely.

2. The device (1) according to claim 1, **characterized in that** the mounting (24) has at least one substantially horizontally aligned contact surface (38) for the filter element (2).

3. The device (1) according to one of the preceding claims, **characterized in that** the mounting (24) is pivotable on the device (1) about a horizontal axis (5), in particular in an angular range α of about ± 5° about the horizontal, preferably of about ± 2° about the horizontal, more preferably of about ± 1° about the horizontal, most preferably of about ± 0.5° about the horizontal.

4. The device (1) according to claim 3, **characterized in that** the mounting (24) is coupled to a drive for driven pivoting in the angular range α about the horizontal.

5. The device (1) according to claim 3 or 4, **characterized in that** the mounting (24) is prestressed in or against the pivoting direction, in particular by means of a spring element.

6. The device (1) according to one of the preceding claims, **characterized in that** the mounting (24) comprises at least one clamping device (25, 26) for holding a filter element (2).

7. The device (1) according to one of the preceding claims, further comprising the substantially cylindrical, replaceable filter element (2), in particular a dialyzer (2).

8. The device (1) according to claim 7, **characterized in that** the filter element (2) has a blood connector (34, 35) and a treatment liquid connector (28, 30) at each of its two end-side sections.

9. The device (1) according to one of the preceding claims, further comprising flexible line sections (27, 29) for connecting the liquid connectors (20, 21, 22, 23) of the internal fluidic system to blood connectors (34, 35) and treatment liquid connectors (28, 30) of the filter element (2).

10. The device (1) according to claim 9, **characterized in that** the liquid connectors (20, 21, 22, 23) of the internal fluidic system are connected or connectable to the flexible line sections (27, 29) and the liquid connectors (20, 21, 22, 23) of the internal fluidic system are positioned relative to the filter element (2) in such a way, and the length of the flexible line sections (27, 29) to be connected to the filter element (2) in each case in such a way that an intended connection of the filter element (2) to the internal fluidic system and/or an intended connection of the liquid connectors (20, 21, 22, 23) to one another is ensured during flushing the internal fluidic system, however, an improper connection of the filter element (2) to the internal fluidic system and/or an improper connection of the liquid connectors (20, 21, 22, 23) to each other during flushing the internal fluidic system is impossible due to an insufficient length of the flexible line sections (27, 29).

## Revendications

1. Dispositif (1) pour le traitement extracorporel de sang, en particulier dispositif de dialyse (1),
avec une fluidique interne pour un fluide de traitement, en particulier pour un fluide de dialyseur,
laquelle fluidique interne présente au moins deux raccordements fluidiques (20, 21, 22, 23) pour raccorder un élément de filtrage (2) essentiellement cylindrique, en particulier un dialyseur (2), à la fluidique interne, afin de guider un fluide de traitement à travers l'élément de filtrage (2), et avec un logement (24) pour un maintien interchangeable de l'élément de filtrage (2) de sorte que l'élément de filtrage (2) puisse être raccordé de façon conforme à sa destination aux raccordements fluidiques (20, 21, 22, 23) de la fluidique interne et à une tubulure de sang (13) extracorporelle,
dans lequel le logement (24) est conçu pour le maintien de l'élément de filtrage (2) de sorte que l'axe longitudinal cylindrique (5) de ce dernier soit orienté essentiellement à l'horizontale et l'élément de filtrage (2) est pivotant dans le logement (24) autour de son axe longitudinal cylindrique (5) de sorte qu'au moins un de ses raccordements fluidiques pointe vers le haut pour soutenir une désaération de l'élément de filtrage lors du remplissage, et/ou en ce qu'au moins un de ses raccordements fluidiques pointe vers le bas pour le vidage de sorte qu'un écoulement de fluide hors de l'élément de filtrage soit facilité et/ou le fluide peut s'écouler ou être aspiré pratiquement en totalité.

2. Dispositif (1) selon la revendication 1, **caractérisé en ce que** le logement (24) présente au moins une surface de contact (38) orientée essentiellement à l'horizontale pour l'élément de filtrage (2).

3. Dispositif (1) selon l'une des revendications précédentes, **caractérisé en ce que** le logement (24) est pivotant autour d'un axe horizontal (5) au niveau du dispositif (1), en particulier dans une plage angulaire α d'environ ± 5° autour de l'horizontale, de préférence d'environ ± 2° autour de l'horizontale, davantage de préférence d'environ ± 1° autour de l'horizontale, particulièrement de préférence d'environ ± 0,5° autour de l'horizontale.

4. Dispositif (1) selon la revendication 3, **caractérisé en ce que** le logement (24) est couplé à un entraînement pour un pivotement entraîné dans la plage angulaire α autour de l'horizontale.

5. Dispositif (1) selon la revendication 3 ou 4, **caractérisé en ce que** le logement (24) est précontraint dans ou à l'encontre de la direction de pivotement, en particulier au moyen d'un élément de ressort.

6. Dispositif (1) selon l'une des revendications précédentes, **caractérisé en ce que** le logement (24) comprend au moins un dispositif de serrage (25, 26) pour le maintien d'un élément de filtrage (2).

7. Dispositif (1) selon l'une des revendications précédentes, qui comprend en outre l'élément de filtrage (2) interchangeable et essentiellement cylindrique, en particulier un dialyseur (2).

8. Dispositif (1) selon la revendication 7, **caractérisé en ce que** l'élément de filtrage (2) présente au niveau de ses deux sections de côté d'extrémité respectivement un raccordement pour le sang (34, 35) et respectivement un raccordement pour le fluide de traitement (28, 30).

9. Dispositif (1) selon l'une des revendications précédentes, qui comprend en outre des sections de tubulure flexibles (27, 29) pour une liaison des raccordements fluidiques (20, 21, 22, 23) de la fluidique interne avec des raccordements pour le sang (34, 35) et des raccordements pour le fluide de traitement (28, 30) de l'élément de filtrage (2).

10. Dispositif (1) selon la revendication 9, **caractérisé en ce que** les raccordements fluidiques (20, 21, 22, 23) de la fluidique interne sont ou peuvent être reliés aux sections de tubulure flexibles (27, 29), et les raccordements fluidiques (20, 21, 22, 23) de la fluidique interne sont positionnés par rapport à l'élément de filtrage (2) de sorte que et la longueur des sections de tubulure flexibles (27, 29) à relier à l'élément de filtrage (2) est respectivement fournie de sorte que un raccordement conforme à sa destination de l'élément de filtrage (2) à la fluidique interne et/ou une liaison conforme à sa destination des raccordements fluidiques (20, 21, 22, 23) ensemble soit assuré(e) lors d'un rinçage de la fluidique interne, alors qu'un raccordement non conforme à sa destination de l'élément de filtrage (2) à la fluidique interne et/ou une liaison non conforme à sa destination des raccordements fluidiques (20, 21, 22, 23) ensemble lors du rinçage de la fluidique interne est impossible du fait d'une longueur insuffisante des sections de tubulure flexibles (27, 29).
